**Europäisches Patentamt**

**European Patent Office**

**Office européen des brevets**

(1) Publication number: **0 109 289**
**B1**

# EUROPEAN PATENT SPECIFICATION

(45) Date of publication of patent specification: **04.11.87**

(21) Application number: **83306917.2**

(22) Date of filing: **11.11.83**

(51) Int. Cl.⁴: **G 01 K 17/00, G 01 N 25/18**

(54) Determining the heat transfer effectiveness of reactors.

(30) Priority: **12.11.82 US 440930**

(43) Date of publication of application:
**23.05.84 Bulletin 84/21**

(45) Publication of the grant of the patent:
**04.11.87 Bulletin 87/45**

(84) Designated Contracting States:
**DE FR GB IT**

(56) References cited:
**EP-A-0 014 934**
**DE-A-3 100 941**
**FR-A-2 484 088**
**GB-A-1 549 841**

(73) Proprietor: **THE BABCOCK & WILCOX COMPANY**
**1010 Common Street P.O. Box 60035**
**New Orleans Louisiana 70160 (US)**

(72) Inventor: **Agarwal, Suresh C.**
**5686 Broadview Road Apt. 2402**
**Parma Ohio 44134 (US)**

(74) Representative: **Cotter, Ivan John et al**
**D. YOUNG & CO. 10 Staple Inn**
**London WC1V 7RD (GB)**

Courier Press, Leamington Spa, England.

## Description

This invention relates to methods of and apparatus for determining the heat transfer effectiveness of reactors.

Reference should be made to our European Patent Applications Nos. 83301958.1 and 83302539.8—Publications Nos. EP 0 092 924 A and EP 0 094 208 A—which relate to the control of reactors and which form part of the state of the art with regard to the present application only within the terms of Article 53, paragraph 4 of the European Patent Convention.

If the heat transfer effectiveness in a reactor, e.g. an olefin oxidation reactor, falls below a selected level, an unsafe or undesirable condition exists so that a measurement of the heat transfer effectiveness can be utilized for fault detection in the reactor.

In an olefin manufacturing process, in particular an ethylene oxide manufacturing process, ethylene and oxygen or air are mixed and fed to an isothermal multitubular reactor. Ethylene is oxidised into ethylene oxide in the presence of a catalyst. Carbon dioxide and water are produced as byproducts.

The aforementioned European Patent Applications Publications Nos. EP 0 092 924 A and EPO 0 094 208 A disclose control schemes for controlling reactor temperature for the exothermic oxidation reaction. This is done by manipulating, among other parameters, the flow rate of coolant based on a maximum and minimum reactor temperature, the heat of reaction and percent oxidation.

During the normal course of heat removal, reactor surfaces become fouled due to the deposition of scale resulting from impurities present in the coolant. This causes changes in the thermal properties of the reactor on the coolant side thereof.

As a consequence, either coolant flow rate must be increased or the surfaces must be cleansed by chemical or manual means. In the absence of these corrective steps, reactor temperature would increase because of a reduced overall heat transfer coefficient. This would result in increased olefin oxidation to by-products, that is, carbon dioxide and water, increased probability of catalyst poisoning, and potential reactor operation in an unsafe region due to the larger heat of reaction of the exothermic side reactions.

It is therefore considered highly desirable or even essential that heat transfer effectiveness be measured and that the results be made available to reactor control system and plant operating personnel so that corrective actions can be taken. It is desirable that the measurement be made "on line" so that a real time measurement of heat transfer effectiveness is provided.

According to the invention there is provided a method of determining the heat transfer effectiveness of a reactor for containing a reaction, the reactor having coolant supply means for supplying a coolant to the reactor, reactant supply means for supplying reactant to the reactor in heat transfer relationship with the coolant over a heat transfer area A, and effluent discharge means for discharging effluent from the reactor, said method comprising:

measuring the coolant flow $F_c$ to the reactor;

providing values corresponding to the heat of vaporisation $\lambda$, the boiling temperature $T_{c_2}$ and the specific heat $C_{p_c}$ of the coolant;

measuring the inlet temperature $T_{c_1}$ of the coolant to the reactor;

determining the log mean temperature difference $\Delta T_m$ across the reactor; and

calculating the actual heat transfer coefficient U of the reactor, which provides a measure of the heat transfer effectiveness thereof, according to the relationship:

$$U = \frac{F_c}{A\Delta T_m} \, [C_{p_c}(T_{c_2}-T_{c_1})+\lambda] \, .$$

The invention also provides apparatus arranged to determine the heat transfer effectiveness of a reactor for containing a reaction, the reactor having coolant supply means for supplying a coolant to the reactor, reactant supply means for supplying reactant to the reactor in heat transfer relationship with the coolant over a heat transfer area A, and effluent discharge means for discharging effluent from the reactor, the apparatus comprising:

coolant flow rate means for measuring the flow rate of coolant $F_c$ to the reactor;

means for providing values corresponding to the heat of vaporisation $\lambda$, the boiling temperature $T_{c_2}$ and the specific heat $C_{p_c}$ of the coolant;

inlet temperature measuring means for measuring the inlet temperature $T_{c_1}$ of the coolant to the reactor;

means for determining the log means temperature difference $\Delta T_m$ across the reactor; and

circuit means arranged to calculate the actual heat transfer coefficient U of the heat transfer area according to the relationship:

$$U = \frac{F_c}{A\Delta T_m} \, [C_{p_c}(T_{c_2}-T_{c_1})+\lambda] \, .$$

2

Preferably, the log mean temperature difference is determined as a function of the reactant temperature entering the reactor, the effluent temperature leaving the reactor, the reaction temperature, the coolant boiling temperature and the coolant inlet temperature. Also preferably, the reaction temperature is obtained by measuring a plurality of temperatures along the height of the reactor, finding a maximum of all the temperature measured along the height of the reactor, finding a maximum of all the temperature measured along the height of the reactor, finding a minimum of all the temperatures measured along the height of the reactor, and obtaining a value between the maximum and minimum temperatures which corresponds to the reaction temperature.

Preferred apparatus embodying the invention and described hereinbelow, for determining the heat transfer effectiveness of a reactor, in particular a reactor for containing an exothermic olefin oxidation reaction, is simple in design, rugged in construction and economical to manufacture.

The invention will now be further described, by way of illustrative and non-limiting example, with reference to the accompanying drawings, in which:

Figure 1 is a schematic representation of an apparatus embodying the invention;

Figure 2 is a schematic representation of another embodiment of the invention; and

Figure 3 is a graph which illustrates a temperature profile for a reaction in a reactor.

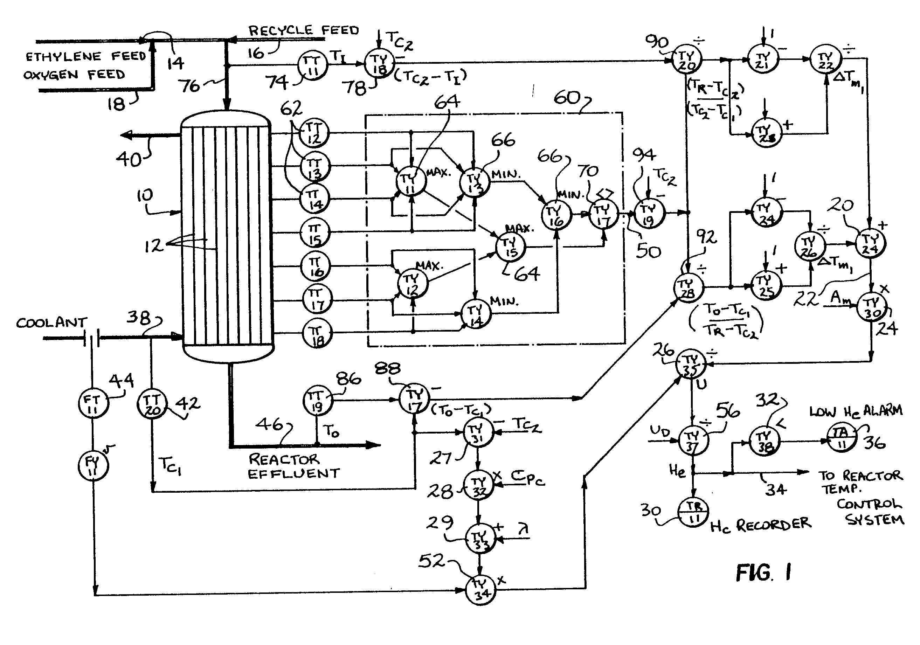

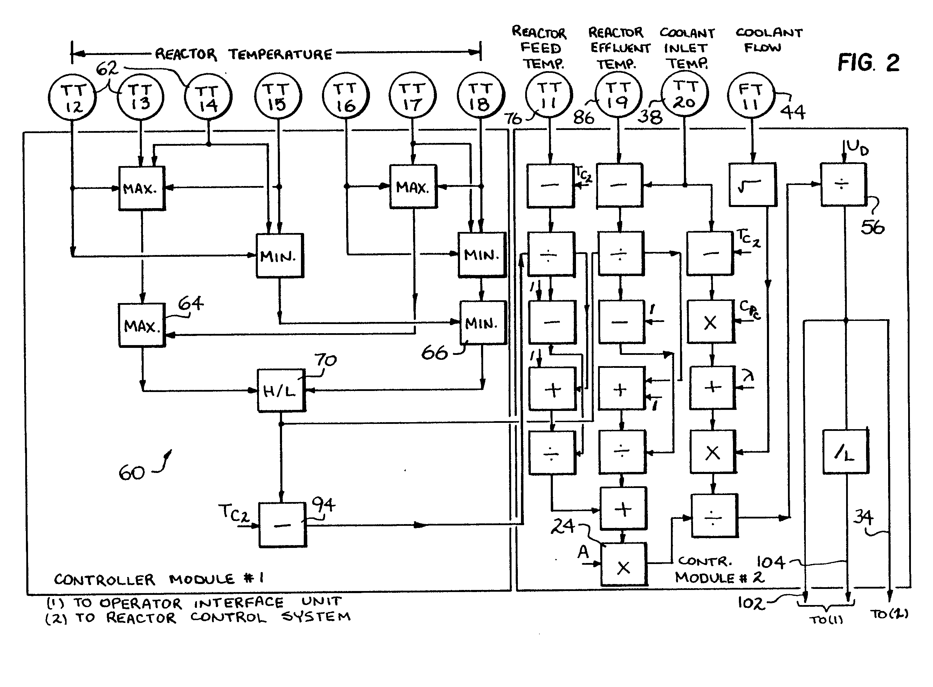

Figures 1 and 2 of the drawings illustrate two separate forms of apparatus embodying the invention for determining the heat transfer effectiveness of a reactor.

Before discussing this apparatus specifically, however, insights and reasoning underlying its implementation are first set forth.

In the following analysis:

$C_{p_c}$=specific heat of a coolant flowing to the reactor;

$F_c$=actual coolant flow rate;

$\lambda$=heat of vaporisation of the coolant;

$T_{c_1}$=inlet temperature of the coolant; and

$T_{c_2}$=boiling temperature of the coolant.

Thus, total heat removed is given by:

$$Q_R = F_c C_{p_c}(T_{c_2} - T_{c_1}) + F_c \lambda \tag{1}$$

Also:

$$Q_R = UA\Delta T_m \tag{2}$$

where:

$Q_R$=total heat rate;

$U$=overall heat transfer coefficient;

$\Delta T_m$=log mean temperature difference; and

$A$=heat transfer area in the reactor.

The heat transfer area A is a fixed value which is available from design data for the reactor. Inlet and outlet temperature are measured for calculating the log mean temperature difference $\Delta T_m$. The boiling temperature $T_{c_2}$ is not measured as it is known from the fluid coolant characteristics.

From equations (1) and (2), there arises the equation:

$$U = \frac{F_c}{A\Delta T_m}[C_{p_c}(T_{c_2} - T_{c_1}) + \lambda] \tag{3}$$

Equation (3) gives the actual heat transfer coefficient for the reactor.

Letting $U_D$ be the overall heat transfer coefficient by reactor design, then the overall heat transfer effectiveness can be defined as:

$$H_e = \frac{U}{U_D} \tag{4}$$

When the heat transfer surface is clean, that is, no fouling exists, then $U_D = U$, and He=1; otherwise $0 \leq H_e < 1$.

Thus, by evaluating the value of $H_e$ only, the actual heat transfer effectiveness can be found, and can be used (a) for compensating coolant flow rate, (b) for generating an alarm for operator information, and (c) in shutdown and emergency control systems for the reactor.

On the reaction side, the temperature profile comprises (a) reaction products at the reaction temperature and (b) cooling of reactor effluent mixture to exit temperature. Consequently, standard methodology for calculation of the log mean temperature difference $\Delta T_m$ is not applicable directly. The following method is used.

3

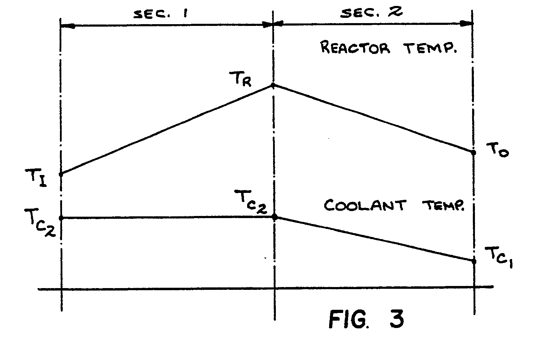

Graphically, temperature profiles may be represented as shown in Figure 3. The overall log mean temperature difference is thus:

$$\Delta T_m = \Delta T_{m_1} + \Delta T_{m_2} \qquad (5)$$

where:

$\Delta T_{m_1}$ = log mean temperature difference for a first section (section 1); and

$\Delta T_{m_2}$ = log mean temperature difference for a second section (section 2).

Using a known relationship for $T_{m_1}$ gives

$$\Delta T_{m_1} = \frac{(T_R - T_{c_2}) - (T_{c_2} - T_l)}{\ln\left(\dfrac{T_R - T_{c_2}}{T_{c_2} - T_l}\right)} \qquad (6)$$

where:

$T_l$ = reaction mixture feed temperature.

By using first order Padé approximation, equation (6) becomes:

$$\Delta T_{m_1} = 2\left[\frac{1 - (T_R - T_{c_2})/(T_{c_2} - T_l)}{1 + (T_R - T_{c_2})/(T_{c_2} - T_l)}\right] \qquad (7)$$

Similarly:

$$\Delta T_{m_2} = 2\left[\frac{1 - (T_o - T_{c_1})/(T_R - T_{c_2})}{1 + (T_o - T_{c_1})/(T_R - T_{c_2})}\right] \qquad (8)$$

where:

$T_o$ = reactor effluent temperature.

Thus, $\Delta T_m$ in equation (3) can be easily calculated from equations (5), (7) and (8).

The embodiment of the invention shown in Figure 1 uses conventional electronic instrument and control components. However, the invention can be easily and naturally implemented in a control computer system as well.

Referring to Figure 1, the reaction temperature $T_R$ is determined from reactor temperature sensors 62 via maximum-minimum logic means 60 and the total log mean temperature difference $\Delta T_m$ is calculated. Thereafter, the actual heat transfer coefficient is calculated from the measured coolant flow rate, coolant inlet temperature, reactor feed and effluent temperature. Finally, a heat transfer effectiveness signal is calculated. The value of this signal is displayed as a trend on a recorder 30, and checked for low level alarm at 32. Also, this signal is available for use in a control system for the reactor over a line 34. An alarm 36 is activated to indicate low level of heat transfer effectiveness and thus a fault in the system.

Bailey 1000 Electronic Analog Instrumentation can be used throughout to provide the measurement of heat transfer effectiveness in the reactor, which is preferably an ethylene oxidate reactor. However, a microprocessor based NETWORK 90 (Trade Mark) Control Computer can also be used for control, alarm, calculation and operator interface.

Figure 2 illustrates an application of the invention with the use of a Network 90 (Trade Mark) Controller Module, product specification E93—906. Operator interface may be provided with digital control stations, product specification E93—902, which are dedicated panel board devices, or by a CRT based Operator Interface Unit, Product specification E93—901, at lines 102 and 104.

Turning once more to Figure 1, the invention embodied therein comprises an apparatus or system for measuring heat effectiveness of a tubular reactor 10 having tubes 12 for the passage of ethylene plus oxygen as a reactant. The reactor 10 is particularly suitable for oxidising ethylene into ethylene oxide with carbon dioxide and water as by-products in the effluent.

Ethylene is provided over a line 14, with recycled ethylene being provided over a line 16. Oxygen or air is provided over a line 18. A reactant inlet line 76, provided with a temperature sensor or transmitter 74, supplies the reactant to the reactor 10 and the effluent is discharged over a line 46 which has a temperature transmitter 86 for measuring the effluent temperature.

Coolant is supplied over a line 38 and is discharged over a line 40. A temperature transmitter 42 and a flow transmitter 44 provide respective temperature and flow values for the coolant fed into the reactor 10.

The maximum and minimum temperatures within the reactor 10 are determined using temperature sensing means and the maximum-minimum logic means 60. The temperature sensing means comprises the individual sensors (or banks of temperature sensors) 62 which are distributed along the length of the reactor 10. Elements 64 are utilised to determine the maximum temperature among the temperature sensors 62 and elements 66 are utilised to determine the minimum temperature along the sensors. Values for minimum and maximum temperature are applied to an element 70, to yield the reaction temperature $T_R$ which appears on a line 50.

The temperature sensor 74 supplies a value corresponding to the reactant input temperature to a subtraction unit 78 which subtracts this quantity from the boiling temperature $T_{c_2}$.

The effluent temperature $T_o$ is provided by the temperature transmitter 86 to a subtraction unit 88 which subtracts the coolant input temperature $T_{c_1}$ therefrom. A subtraction element 94 effects subtraction of the coolant boiling temperature from the reaction temperature. Division elements 90 and 92 cooperate with the subtraction element 94 to respectively generate major terms in Equations (7) and (8) above.

These equations are completed in the logic elements shown in Figure 1 to the right of the division elements 90 and 92, which logic elements each have the function shown and cooperate with a final addition element 20 to generate, on a line 22, the log mean temperature difference $\Delta T_m$. This is multiplied by the heat transfer area A by the multiplication element 24 and supplied to a division element 26.

The constant factors corresponding respectively to coolant boiling temperature, coolant specific heat and coolant heat of vaporisation are factored in by subtraction, multiplication, and addition elements 27, 28 and 29. These elements cooperate with a multiplication unit 52 which factors in the coolant flow value and cooperates with the division element 26 to complete Equation (3) above.

The overall heat transfer effectiveness $H_e$ is determined and applied to the line 34 by a division element 56 that factors in the design heat transfer coefficient $U_D$.

Referring to Figure 2, the same function can be achieved utilising the Network 90 circuitry. Specifically, a control module 1 and a control module 2 are utilised as shown in Figure 2 to achieve the various mathematical and logic functions. Similar parts having similar functions to parts of Figure 1 are designated with the same numerals as in Figure 1. Since the functioning is the same, no additional details are provided for Figure 2.

**Claims**

1. A method of determining the heat transfer effectiveness of a reactor for containing a reaction, the reactor 10 having coolant supply means (38) for supplying a coolant to the reactor, reactant supply means (76) for supplying reactant to the reactor in heat transfer relationship with the coolant over a heat transfer area A, and effluent discharge means (46) for discharging effluent from the reactor, said method comprising:

measuring (44) the coolant flow $F_c$ to the reactor (10);

providing values corresponding to the heat of vaporisation $\lambda$, the boiling temperature $T_{c_2}$ and the specific heat $C_{p_c}$ of the coolant;

measuring (42) the inlet temperature $T_{c_1}$ of the coolant to the reactor;

determining the log mean temperature difference $\Delta T_m$ across the reactor; and

calculating the actual heat transfer coefficient U of the reactor, which provides a measure of the heat transfer effectiveness thereof, according to the relationship:

$$U= \frac{F_c}{A\Delta T_m} [C_{p_c}(T_{c_2}-T_{c_1})+\lambda] .$$

2. A method according to claim 1, including providing a value corresponding to a desired design heat transfer coefficient $U_D$, and calculating the overall heat transfer effectiveness $H_e$ for the reactor (1) according to the relationship:

$$H_e=\frac{U}{U_D} .$$

3. A method according to claim 2, including measuring the value of the heat transfer effectiveness $H_e$, comparing (32) the measured heat transfer effectiveness $H_e$ to a lower limit for the heat transfer effectiveness of the reactor below which a fault exists in the reactor, and activating an alarm (36) when the measured effectiveness falls below the lower limit therefor.

4. A method according to claim 1, claim 2 or claim 3, including measuring (74) the temperature $T_I$ of reactant entering the reactor (10), measuring (86) the temperature $T_o$ of effluent leaving the reactor, measuring the reaction temperature $T_R$ of the reaction in the reactor, and calculating the log mean temperature difference $\Delta T_m$ as a function of the reactant, effluent and reaction temperatures.

5. A method according to claim 4, including calculating the log mean temperature difference of the reactor (10) according to the equation:

$$\Delta T_m=2\left[\frac{1-(T_R-T_{c_2})/(T_{c_2}-T_I)}{1+(T_R-T_{c_2})/(T_{c_2}-T_I)}\right] +2\left[\frac{1-(T_o-T_{c_1})/(T_R-T_{c_2})}{1+(T_o-T_{c_1})/(T_R-T_{c_2})}\right]$$

5

6. A method according to any one of the preceding claims, wherein the reactor contains an exothermic olefin oxidation reaction.

7. A method according to claim 6, wherein the reactor contains an ethylene oxidation reaction.

8. Apparatus arranged to determine the heat transfer effectiveness of a reactor for containing a reaction, the reactor (10) having coolant supply means (38) for supplying a coolant to the reactor, reactant supply means (76) for supplying reactant to the reactor in heat transfer relationship with the coolant over a heat transfer area A, and effluent discharge means (46) for discharging effluent from the reactor, the apparatus comprising:

coolant flow rate means (44) for measuring the flow rate of coolant $F_c$ to the reactor (10);

means for providing values corresponding to the heat of vaporisation $\lambda$, the boiling temperature $T_{c2}$ and the specific heat $C_{p_c}$ of the coolant;

inlet temperature measuring means (42) for measuring the inlet temperature $T_{c1}$ of the coolant to the reactor;

means for determining the log means temperature difference $\Delta T_m$ across the reactor; and

circuit means arranged to calculate the actual heat transfer coefficient U of the heat transfer area according to the relationship:

$$U = \frac{F_c}{A\Delta T_m} [C_{p_c}(T_{c2} - T_{c1}) + \lambda] .$$

9. Apparatus according to claim 8, wherein the means for determining the log mean temperature difference comprises reaction temperature determining means connected to the reactor for determining the reaction temperature $T_R$, reactant inlet temperature measuring means (74) for measuring the inlet temperature $T_I$ of the reactant, effluent temperature measuring means (86) for measuring the effluent temperature $T_o$ leaving the reactor, and a circuit for calculating the log mean temperature difference .according to the relationship:

$$\Delta T_m = 2[\frac{1-(T_R-T_{c2})/(T_{c2}-T_I)}{1+(T_R-T_{c2})/(T_{c2}-T_I)}] + 2[\frac{1-(T_o-T_{c1})/(T_R-T_{c2})}{1+(T_o-T_{c1})/(T_R-T_{c2})}]$$

10. Apparatus according to claim 9, which includes a plurality of temperature transmitters (62) connected at spaced locations along a length of the reactor (10), maximum temperature means (64) connected to each of the temperature transmitters (62) for establishing maximum temperature measured thereby, minimum temperature means (66) connected to each of the temperature transmitters (62) for establishing the minimum temperature measured thereby, and reaction temperature means (70) connected to the minimum and maximum circuit means (64, 66) for establishing a reaction temperature between the minimum and maximum temperatures.

11. Apparatus according to claim 8, claim 9 or claim 10, including means for providing a value corresponding to a desired design heat transfer coefficient $U_D$, and means (56) for establishing a heat transfer effectiveness (He) of the reactor according to the relationship:

$$H_e = \frac{U}{U_D} .$$

12. Apparatus according to claim 11, including comparison means (32) for comparing a calculated actual heat transfer effectiveness He to a lower limit for heat transfer effectiveness of the reactor (10) below which a fault exists, and alarm means (36) connected to the comparator means (32) for activating an alarm when the actual heat transfer effectiveness falls below the lower limit therefor.

**Patentansprüche**

1. Verfahren zum Bestimmen der Wärmeübertragungseffektivität eines Reaktors bzw. Reaktorgefäßes, in welchem eine Reaktion ablaufen soll, wobei der Reaktor (10) Kühlmittelzufuhreinrichtungen (38) zum Zuführen eines Kühlmittels zu dem Reaktor, Reaktionsmittelzuführeinrichtungen (76) zum Zuführen von Reaktionsmittel zu dem Reaktor in Wärmeaustauschbeziehung mit dem Kühlmittel über einen Wärmeaustauschbereich A und gereinigte Abwasser-Abflußeinrichtungen (46) zum Abfließen gereinigten Abwassers aus dem Reaktor hat, wobei das Verfahren aufweist:

Messen (44) des Kühlmittelflusses $F_c$ zu dem Reaktor (10);

Vorsehen von Werten, welche der Verdampfungswärme $\lambda$, der Siedetemperatur $T_{c2}$ und der spezifischen Wärme $C_{p_c}$ des Kühlmittels;

Messen (42) der Einlaßtemperatur $T_{c1}$ des Kühlmittels zu dem Reaktor;

Bestimmen der registrierten mittleren Temperaturdifferenz $\Delta T_m$ über den Reaktor; und

Berechnen des tatsächlichen Wärmeaustauschkoeffizienten U des Reaktors, welcher einen Maßstab für dessen Wärmeaustauscheffektivität bildet und zwar gemäß der Gleichung

$$U = \frac{F_c}{A\Delta T_m} \; [C_{Pc}(T_{c2} - T_{c1}) + \lambda] \; .$$

2. Verfahren nach Anspruch 1, wobei eingeschlossen ist, einen Wert entsprechend einem gewünschten Konstruktionswärmeaustauschkoeffizienten $U_D$ vorzusehen und die gesamte Wärmeaustauscheffektivität $H_e$ für den Reaktor (1) auszurechnen gemäß der Gleichung:

$$H_e = \frac{U}{U_D} \; .$$

3. Verfahren gemäß Anspruch 2, wobei eingeschlossen ist, den Wert der Wärmeaustauscheffektivität $H_e$ zu messen, die gemessene Wärmeaustauscheffektivität $H_e$ zu vergleichen (32) mit einer unteren Grenze für die Wärmeaustauscheffektivität des Reaktors, unterhalb derer ein Fehler in dem Reaktor vorliegt, und einen Alarm (36) auszulösen, wenn die gemessene Effektivität unter den unteren Grenzwert dafür fällt.

4. Verfahren nach Anspruch 1, Anspruch 2 oder Anspruch 3, wobei eingeschlossen ist, die Temperatur $T_1$ des Reaktionsmittels, welches in den Reaktor (10) eintritt, zu messen, die Temperatur $T_o$ des gereinigten Abwassers, welches den Reaktor verläßt, zu messen, die Reaktionstemperatur $T_R$ der Reaktion in dem Reaktor zu messen und die registrierte mittlere Temperaturdifferenz $\Delta T_m$ zu berechnen der Reaktionsmittel-, Abwasser- und Reaktionstemperaturen.

5. Verfahren nach Anspruch 4, welches das Bereichnen der registrierten mittleren Temperaturdifferenz des Reaktors (10) gemäß der Gleichung einschließt:

$$\Delta T_m = 2 \left[\frac{1 - (T_R - T_{c2})/(T_{c2} - T_l)}{1 + (T_R - T_{c2})/(T_{c2} - T_l)}\right] + 2 \left[\frac{1 - T_o - T_{c1})/(T_R - T_{c2})}{1 + (T_o - T_{c1})/(T_R - T_{c2})}\right]$$

6. Verfahren nach einem der vorstehenden Ansprüche, wobei in dem Reaktor eine exotherme Olefinoxidationsreaktion abläuft.

7. Verfahren nach Anspruch 6, wobei in dem Reaktor eine Ethylenoxidationsreaktion abläuft.

8. Vorrichtung, welche zum Bestimmen der Wärmeaustauscheffektivität eines Reaktors ausgelegt ist, in welchem eine Reaktion abläuft, wobei der Reaktor (10) Kühlmittelzufuhreinrichtungen (38) zum Zuführen eines Kühlmittels zu dem Reaktor, Reaktionsmittelzuführeinrichtungen (76) zum Zuführen von Reaktionsmittel zu dem Reaktor in Wärmeaustauschbeziehung mit dem Kühlmittel über eine Wärmeaustauschfläche A und Abwasserauslaßeinrichtungen (46) für das Entleeren von Abwasser aus dem Reaktor hat, wobei die Vorrichtung aufweist:

eine Kühlmittelflußrateneinrichtung (44) zum Messen der Flußrate $F_c$ (des Durchesatzes) zu dem Reaktor (10);

Einrichtungen, um Werte vorzusehen, welche der Verdampfungswärme $\lambda$, der Siedetemperatur $T_{c2}$ und der spezifischen Wärme $C_{Pc}$ des Kühlmittels entsprechen;

eine Einlaßtemperaturmeßeinrichtung (42) zum Messen der Einlaßtemperatur $T_{c1}$ des Kühlmittels zu dem Reaktor;

eine Einrichtung zum Bestimmen der registrierten mittleren Temperaturdifferenz der $\Delta T_m$ über den Reaktor; und

eine Schaltkreiseinrichtung, welche zum Berechnen des tatsächlichen Wärmeaustauschkoeffizienten U der Wärmeaustauschfläche ausgelegt ist gemäß der Beziehung:

$$U = \frac{F_c}{A\Delta T_m} \; [C_{Pc}(T_{c2} - T_{c1}) + \lambda] \; .$$

9. Vorrichtung nach Anspruch 8, wobei die Einrichtung zum Bestimmen der registrierten mittleren Temperaturdifferenz aufweist: eine Reaktionstemperaturbestimmungseinrichtung, welche mit dem Reaktor verbunden ist, zum Bestimmen der Reaktionstemperatur $T_R$, eine Reaktionsmitteleinlaßtemperatur-Meßeinrichtung (74) zum Messen der Einlaßtemperatur $T_1$ des Reaktionsmittels, eine Abwassertemperatur-Meßeinrichtung (86) zum Messen der Temperatur $T_o$ des Abwassers, welches den Reaktor verläßt, und einen Schaltkreis zum Bereichnen der registrierten mittleren Temperaturdifferenz gemäß der Gleichung:

$$\Delta T_m = 2 \left[\frac{1 - (T_R - T_{c2})/(T_{c2} - T_l)}{1 + (T_R - T_{c2})/(T_{c2} - T_l)}\right] + 2 \left[\frac{1 - (T_o - T_{c1})/(T_R - T_{c2})}{1 + (T_o - T_{c1})/(T_R - T_{c2})}\right]$$

**0 109 289**

10. Vorrichtung nach Anspruch 9, welche einschließt: eine Vielzahl von Temperaturmeldern (62), welche an voneinander beabstandeten Stellen entlang der Länge des Reaktors (10) angeschlossen sind, Maximaltemperatureinrichtungen (64), welche mit jedem der Temperaturmelder (62) verbunden sind, zum Festlegen der durch diese gemessenen Maximaltemperatur, Minimaltemperatureinrichtungen (66), welche mit jedem der Temperaturmelder (62) verbunden sind, zum Festlegen der durch diese gemessenen Minimaltemperatur und Reaktionstemperatureinrichtungen (70), welche mit den Minimal- und Maximalschaltkreiseinrichtungen (64, 66) verbunden sind, zum Festlegen einer Reaktionstemperatur zwischen den Minimal- und Maximaltemperaturen.

11. Vorrichtung nach Anspruch 8, Anspruch 9 oder Anspruch 10, welcher einschließt: eine Einrichtung zum Vorsehen eines Wertes, welcher einem gewünschten Konstruktonswärmeaustauschkoeffizienten $U_D$ entspricht, und eine Einrichtung (56) zum Festlegen einer Wärmeaustauscheffektivität ($H_e$) des Reaktors gemäß der Gleichung:

$$H_e = \frac{U}{U_D} \; .$$

12. Vorrichtung gemäß Anspruch 11, welche einschließt: eine Vergleichseinrichtung (32) zum Vergleichen einer berechneten tatsächlichen Wärmeaustauscheffektivität $H_e$ mit einem unteren Grenzwert für die Wärmeaustauscheffektivität des Reaktors (10), unterhalb derer ein Fehler vorliegt, und eine Alarmeinrichtung (36), welcher an die Vergleichseinrichtung (32) angeschlossen ist, zum Auslösen eines Alarms, wenn die tatsächliche Wärmeaustauscheffektivität unter ihren unteren Grenzwert fällt.

**Revendications**

1. Un procédé de détermination de l'efficacité du transfert de chaleur d'un réacteur devant être le siège d'une réaction, ce réacteur (10) comportant des moyens d'alimentation en fluide de refroidissement (38) destinés à introduire un fluide de refroidissement dans le réacteur, des moyens d'alimentation en substances devant réagir (76) destinés à introduire dans le réacteur des substances devant réagir, en relation de transfert de chaleur avec le fluide de refroidissement sur une aire de transfert de chaleur A, et des moyens d'évacuation d'effluent (46) destinés à évacuer un effluent hors du réacteur, ce procédé comprenant les opérations suivantes:

on mesure (44) le débit de fluide de refroidissement $F_c$ entrant dans le réacteur (10);

on fournit des valeurs correspondant à la chaleur de vaporisation $\lambda$, à la température d'ébullition $T_{c_2}$ et à la chaleur spécifique $C_{p_c}$ du fluide de refroidissement;

on mesure (42) la température d'entrée $T_{c_1}$ du fluide de refroidissement dans le réacteur;

on détermine la différence de température logarithmique moyenne $\Delta T_m$ de part et d'autre du réacteur; et

on calcule le coefficient de transfert de chaleur réel U du réacteur, ce qui procure une mesure de l'efficacité du transfert de chaleur de ce réacteur, conformément à la relation:

$$U = \frac{F_c}{A \Delta T_m} \; [C_{p_c}(T_{c_2} - T_{c_1}) + \lambda]$$

2. Un procédé selon la revendication 1, dans lequel on fournit une valeur correspondant à un coefficient de transfert de chaleur nominal désiré $U_D$, et on calcule l'efficacité du transfert de chaleur global $H_e$ pour le réacteur (1) conformément à la relation:

$$H_e = \frac{U}{U_D}$$

3. Un procédé selon la revendication 2, comprenant la mesure de la valeur de l'efficacité du transfert de chaleur $H_e$, la comparaison (32) de l'efficacité du transfert de chaleur mesurée $H_e$ avec une limite inférieure de l'efficacité du transfert de chaleur du réacteur, au-dessous de laquelle une condition de défaut est présente dans le réacteur, et l'activation d'une alarme (36) lorsque l'efficacité mesurée tombe au-dessous de sa limite inférieure.

4. Un procédé selon la revendication 1, la revendication 2 ou la revendication 3, comprenant la mesure (74) de la température $T_i$ des substances devant réagir qui entrent dans le réacteur (10), la mesure (86) de la température $T_o$ de l'effluent qui sort du réacteur, la mesure de la température de réaction $T_R$ de la réaction qui se déroule dans le réacteur, et le calcul de la différence de température logarithmique moyenne $\Delta T_m$ en fonction des températures des substances devant réagir, de l'effluent et de réaction.

5. Un procédé selon la revendication 4, dans lequel on calcule la différence de température logarithmique moyenne du réacteur (10) conformément à l'équation:

8

**0 109 289**

$$\Delta T_m = 2 \; \left[\frac{1-(T_R-T_{c2})/(T_{c2}-T_I)}{1+(T_R-T_{c2})/(T_{c2}-T_I)}\right] + 2 \; \left[\frac{1-(T_o-T_{c2})/(T_R-T_{c2})}{1+(T_o-T_{c1})/(T_R-T_{c2})}\right]$$

6. Un procédé selon l'une quelconque des revendications précédentes, dans lequel le réacteur est le siège d'une réaction d'oxydation d'oléfine, qui est une réaction exothermique.

7. Un procédé selon la revendication 6, dans lequel le réacteur est le siège d'une réaction d'oxydation d'éthylène.

8. Dispositif conçu pour déterminer l'efficacité du transfert de chaleur d'un réacteur devant être le siège d'une réaction, ce réacteur (10) comportant des moyens d'alimentation en fluide de refroidissement (38) destinés à introduire un fluide de refroidissement dans le réacteur, des moyens d'alimentation en substances devant réagir (76) destinés à introduire dans le réacteur des substances devant réagir, en relation de transfert de chaleur avec le fluide de refroidissement sur une aire de transfert de chaleur A, et des moyens d'évacuation d'effluent (46) destinés à évacuer un effluent hors du réacteur, le dispositif comprenant:

des moyens de mesure de débit de fluide de refroidissement (44) destinés à mesurer le débit du fluide de refroidissement $F_c$ entrant dans le réacteur (10);

des moyens destinés à fournir des valeurs correspondant à la chaleur de vaporisation $\lambda$, à la température d'ébullition $T_{c2}$ et à la chaleur spécifique $C_{pc}$ du fluide de refroidissement;

des moyens de mesure de température d'entrée (42) destinés à mesurer la température d'entrée $T_{c1}$ du fluide de refroidissement qui est introduit dans le réacteur;

des moyens destinés à déterminer la différence de température logarithmique moyenne $\Delta T_m$ de part et d'autre du réacteur; et

un circuit conçu pour calculer le coefficient de transfert de chaleur réel U de l'air de transfert de chaleur conformément à la relation:

$$U = \frac{F_c}{A\Delta T_m} \; [C_{pc}(T_{c2}-T_{c1})+\lambda]$$

9. Dispositif selon la revendication 8, dans lequel les moyens destinés à déterminer la différence de température logarithmique moyenne comprennent des moyens de détermination de température de réaction, connectés au réacteur de façon à déterminer la température de réaction $T_R$, des moyens de mesure de la température d'entrée des substances devant réagir (74), destinés à mesurer la température d'entrée $T_I$ des substances devant réagir, des moyens de mesure de température d'effluent (86) destinés à mesurer la température de l'effluent $T_o$ qui sort du réacteur, et un circuit destiné à calculer la différence de température logarithmique moyenne conformément à la relation:

$$\Delta T_m = 2 \; \left[\frac{1-(T_R-T_{c2})/(T_{c2}-T_I)}{1+(T_R-T_{c2})/(T_{c2}-T_I)}\right] + 2 \; \left[\frac{1-(T_o-T_{c2})/(T_R-T_{c2})}{1+(T_o-T_{c1})/(T_R-T_{c2})}\right]$$

10. Dispositif selon la revendication 9, comprenant un ensemble d'émetteurs de température (62) disposés en des positions espacées sur une longueur du réacteur (10), des moyens de détermination de température maximale (64) connectés à chacun des émetteurs de température (62) pour déterminer la température maximale mesurée par ces derniers, des moyens de détermination de température minimale (66) connectés à chacun des émetteurs de température (62) pour déterminer la température minimale mesurée par ces derniers, et des moyens de détermination de température de réaction (70) connectés aux moyens de détermination de température minimale et de température maximale (64, 66), pour déterminer une température de réaction comprise entre les températures minimale et maximale.

11. Dispositif selon la revendication 8, la revendication 9 ou la revendication 10, comprenant des moyens destinés à fournir une valeur correspondant à un coefficient de transfert de chaleur nominal désiré $U_D$, et des moyens (56) destinés à déterminer l'efficacité du transfert de chaleur $H_e$ du réacteur conformément à la relation:

$$H_e = \frac{U}{U_D}$$

12. Dispositif selon la revendication 11, comprenant des moyens des comparaison (32) destinés à comparer une efficacité de transfert de chaleur réelle calculée $H_e$ avec une limite inférieure pour l'efficacité du transfert de chaleur de réacteur (10), au-dessous de laquelle une condition de défaut existe, et des moyens d'alarme (36) connectés aux moyens de comparaison (32) de façon à activer une alarme lorsque l'efficacité de transfert de chaleur réelle tombe au-dessous de sa limite inférieure.

FIG. 1

FIG. 2

REACTOR TEMPERATURE

REACTOR FEED TEMP.

REACTOR EFFLUENT TEMP.

COOLANT INLET TEMP.

COOLANT FLOW

CONTROLLER MODULE #1

CONTR. MODULE #2

(1) TO OPERATOR INTERFACE UNIT
(2) TO REACTOR CONTROL SYSTEM

0 109 289

FIG. 3